Europäisches Patentamt

European Patent Office

Office européen des brevets

(11) Publication number: **0 289 666 B1**

(12) **EUROPEAN PATENT SPECIFICATION**

(45) Date of publication of patent specification: **17.06.92**   (51) Int. Cl.5: **C12N 5/02**, C02F 3/10,
                                                                                        C12M 3/04

(21) Application number: **87302951.6**

(22) Date of filing: **03.04.87**

(54) **Cell culture carriers etc.**

(43) Date of publication of application:
   **09.11.88 Bulletin 88/45**

(45) Publication of the grant of the patent:
   **17.06.92 Bulletin 92/25**

(84) Designated Contracting States:
   **AT BE CH DE ES FR GR IT LI LU NL SE**

(56) References cited:
   **EP-A- 0 021 257**
   **US-A- 3 740 321**
   **US-A- 3 853 712**

(73) Proprietor: **YEDA RESEARCH AND DEVELOP-
   MENT COMPANY LIMITED
   P.O. Box 95
   Rehovot 76100(IL)**

(72) Inventor: **Bohak, Zvi
   26 Moscovitz Street
   Rehovot(IL)**
   Inventor: **Kadouri, Avinoam
   12 Shapira Street
   Petach Tikva(IL)**
   Inventor: **Maroudas, George Nicholas
   71 Park Avenue North
   London(GB)**

(74) Representative: **Dodd, David Michael et al
   ROYSTONS 531 Tower Building Water Street
   Liverpool L3 1BA(GB)**

**Description**

Most cells, including normal tissue derived primary cells, are anchorage-dependent, and require a solid surface in order to proliferate, metabolize nutrients and produce biomolecules. Conventional means for growth of these cells on a small scale include petri-dishes, flasks and roller bottles. A number of approaches have been taken by various investigators to develop cell reactors with large surface-area-to-volume ratios for space and operational economy. Such devices include polymer sponge matrices, multiple tubing, stacked-plate systems, coiled plastic sheets, microcarrier suspension culture and hollow-fibres. Another approach is to culture cells on a packing material inside a column shaped vessel. One of these systems uses glass beads designed for large scale cell cultivation. Another packing material has a "saddle" shape.

The packing substratum must have several properties:

(i) To enable the attached cells to spread to their optimal size and shape in order to metabolise and proliferate;

(ii) To promote exchange of metabolites between the cells and the culture medium, by providing an increased ratio of surface to volume inside the packed vessel;

(iii) To facilitate medium flow to the cells by providing an increased ratio of surface to volume inside the packed vessel;

(iv) To maintain high cell density for long periods, for the production of various biomolecules, even in serum free media.

According to this invention there is provided a packing material element for use in bulk cultivation of anchorage-dependent cells, characterised in that the element comprises a length of plastics material formed by twisting a plane sheet or ribbon about a longitudinal axis. Such elements, which can be randomly packed, can be fabricated from plastics polymers, preferably transparent, such as polystyrene, polypropylene, and polyester.

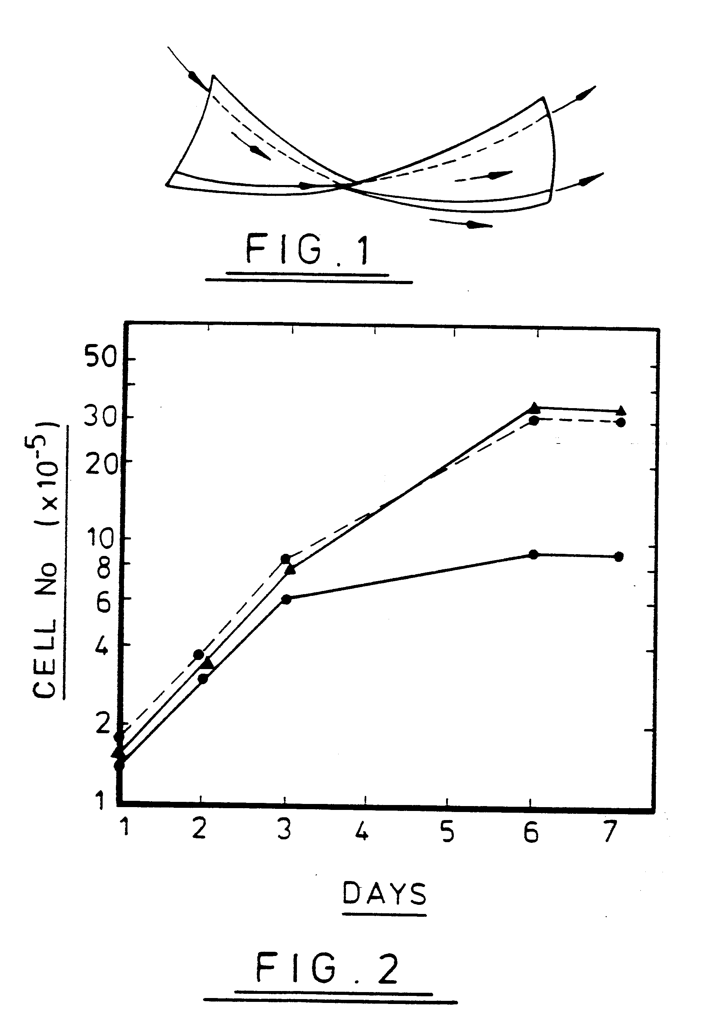

The specific geometry of the packing material can be obtained by rotary deformation of a plane sheet or ribbon, for example the linear twisted planar helix (Fig 1). This consists of a length of flat ribbon which has been twisted into a helical shape around its longer axis. Three advantages results from this geometry in cell culture:

(i) Improved spreading and alignment of cells due to the predominatly flat, curved plane geometry.

(ii) Improved physiocochemical hydrodynamics, due to the helical flow pattern which is both open and twisted (Fig 1). The flat planar geometry enables easy viewing of cells (if the polymer is transparent).

The planar packing elements of the present invention are superior to solid spheres in the following respects:

i. Larger areas for cells to spread, for equal mass of packing material;

ii. Larger surface for mass transfer for equal volumes of packing elements;

iii. Better hydrodynamic flow and mixing;

iv. Less weight for equal surface area of packing material due to low solid volume;

v. Greater transparency, lower optical refraction due to thinner and planar surfaces;

vi. It can be easily sampled for microscopic examination and for determination of cell numbers;

vii. Packing elements are readily made from plastics films or ribbon by standard techniques of hot pressing, stamping, rolling, twisting and cutting. Alternatively, the same shape can be cut from continuous extrusions.

Suitable plastics are polystyrene, polypropylene and polyester which can be surface treated for cell adhesion, by various methods well known in the literature.

Suitable dimensions of these elements are: Film thickness 0.05mm to 0.25mm, length of each unit 2mm or greater, width 1mm to 5mm.

Such planar packing units are suitable mainly for use as filling of column type reactors with medium perfusion. They can also be used as packing in roller bottles.

In practical implementation of the invention, any tendency for nesting of packing elements can be countered by appropriate installation with or without agitation or other attitude adjustment/variation and/or by assuring variety of precise geometry.

Examples

The materials used for cell culture were passed through a discharge from a plasma torch then washed with methanol, dried and sterilized under UV light. Before cells seeding the carrier was immersed in a solution of 100 ug/ml of poly-D-lysine and rinsed with sterile water.

2

1. Twisted squares and ribbons of polystyrene (and by way of comparison cylindrical elements having the same computed surface area) were placed with Dulbecco's modified Eagle's medium (DMEM) supplemented with 10%, fetal calf serum (FCS) in a test tube and seeded with the same number of cells. 24 hrs after seeding, cell adherence was determined by tyrpsinization. (Table 1).

2. Polystyrene twisted ribbons (and by way of comparison polystyrene cylinders) having a surface area of about 60 cm were placed in test tubes and covered with culture medium. They were seeded with $1.5 \times 10^5$ cells and left overnight at 37 C to allow the cells to attach. The pre-seeded supports were then transferred to a siliconized petri-dish and cell proliferation was monitored. Tissue culture plates were used as control. Cells reached $5-6 \times 10^4$ cells/cm , the same surface density as obtained on petri-dishes (Fig 2). In Figure 2 the upper continous line represents twisted ribbon, the broken line represents cylinders and the lower continuous line represents the control.

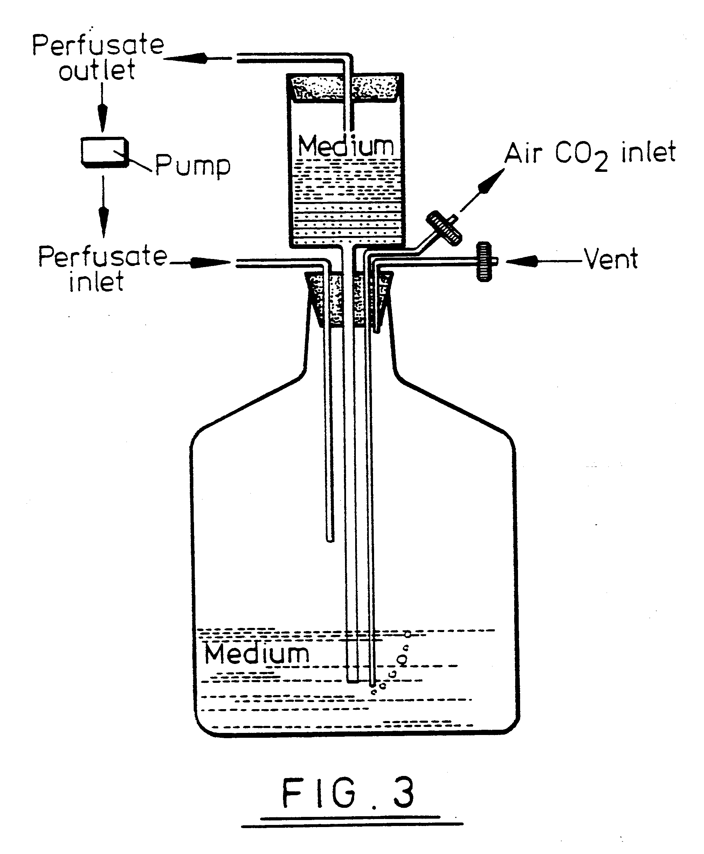

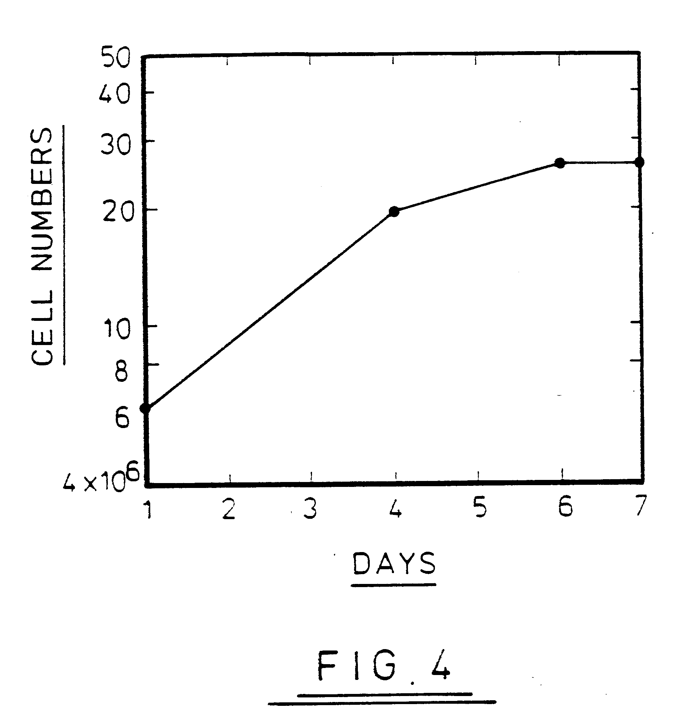

3. Cell proliferation on polystyrene twisted ribbons (and, by way of comparison polystyrene cylinders having the same surface area) was tested in a stationary column reactor. A laboratory-scale reactor assembly is depicted in Fig 3. The carrier was placed in plastic test tubes, covered with culture medium and seeded ih human diploid fibroblasts cells. The tubes were rotated 1/3 turn every 5 min to allow the cells to settle and spread evenly over both sides of the carrier units. After 18 hrs the carrier with the attached cells was transferred to the column reactor. The carrier was covered with medium. The reservoir was then filled with 2 medium and medium circulation was started. Pump speed was adjusted to give an initial medium residence time of 20 mins. As the cells proliferated, pumping rate was increased to decrease residence time to 5 min. The reactor chamber was sampled periodically by taking out pieces of the cell carrier for cells number determination (Fig 4). At confluency cell density reached $5-6 \times 10^4$ cells/cm and from $0.7 \times 10^6$ to $2 \times 10^6$ per ml of reactive volume, depending on the density of the packing material.

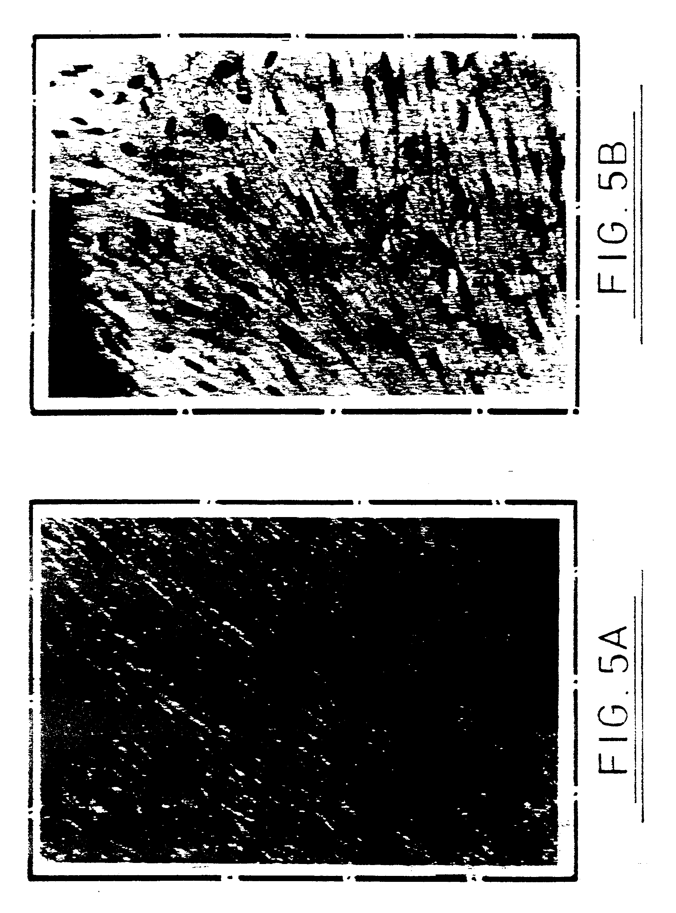

4. Phase-contrast microscopy of a carrier sample taken from the above reactor showed that cells settle spread and grow evenly over both sides of the carrier (Fig 5).

5. Polystyrene twisted ribbons (and, by way of comparison polystyrene cylinders) having a calculated surface area of 2900 cm$^2$ were packed into 100 ml glass roller bottle. $1 \times 10^7$ cells were seeded and the bottle was half filled with medium. The growth medium was replaced daily to avoid nutrient exhaustion. The cell proliferation was followed by removing periodically pieces of carrier and counting the cells after trypsinization. Cells proliferated with an average doubling time of 1.2 to 1.3 days and reached constant level of $3.4 \times 10^4$ cells/cm$^2$ or carrier.

Table 1

| Fibroblast adherences to polystyrene particles of various physical shapes | |
|---|---|
| Physical shape | Cells adhered 24 hrs after plating (per cent) * |
| Short cylinders | 85 |
| Open cylinder segments | 90 |
| Cylindrical planar helix | 90 |
| Twisted Squares | 95 |
| Twisted ribbons | 98 |

\* Percent of control - Tissue culture perti-dishes

## Claims

1. A packing material element for use in bulk cultivation of anchorage-dependent cells characterised in that the element comprises a length of plastics material formed by twisting a plane sheet or ribbon about a longitudinal axis.

2. An element according to claim 1 in the form of a length of ribbon twisted to form a linear planar helix.

3. An element according to claim 1 in the form of a twisted square.

4. An element according to any of the preceding claims characterised in that the plastics material is transparent.

5. An element according to any of the preceding claims characterised in that the plastics material is polystyrene, polypropylene or a polyester.

6. An element according to any of the preceding claims characterised in that the element has a thickness of from 0.05mm to 0.25mm, a length of at least 2mm and a width of from 1mm to 5mm.

7. A method of bulk cultivation of anchorage-dependent cells in a vessel containing packing material elements characterised in that the said elements are elements as claimed in any of the preceding claims.

8. A method of cultivation according to claim 7 characterised in that the packing elements are randomly packed.

9. A method according to claim 7 or claim 8 characterised in that the cultivation is carried out in a column-type reactor with medium perfusion.

10. A method according to claim 7 or claim 8 characterised in that the cultivation is carried out in roller bottles.

**Revendications**

1. Un élément de matériau de remplissage utilisable pour la culture en volume de cellules dépendant de l'ancrage,
caractérisé en ce que l'élément comprend une longueur de matière plastique formée en tordant une feuille ou un ruban plan autour d'un axe longitudinal.

2. Un élément selon la revendication 1, sous forme d'une longueur de ruban tordue pour former une hélice planaire linéaire.

3. Un élément selon la revendication 1, sous forme d'un carré tordu.

4. Un élément selon l'une quelconque des revendications précédentes,
caractérisé en ce que la matière plastique est transparente.

5. Un élément selon l'une quelconque des revendications précédentes,
caractérisé en ce que la matière plastique est du polystyrène, du polypropylène ou un polyester.

6. Un élément selon l'une quelconque des revendications précédentes,
caractérisé en ce que l'élément a une épaisseur allant de 0,05mm à 0,25mm, une longueur d'au moins 2mm et une largeur allant de 1mm à 5mm.

7. Un procédé de culture en volume de cellules dépendant de l'ancrage dans un récipient contenant des éléments de matériau de remplissage,
caractérisé en ce que lesdits éléments sont des éléments revendiqués dans l une quelconque des revendications précédentes.

8. Un procédé de culture selon la revendication 7,
caractérisé en ce que les éléments de remplissage sont remplis au hasard.

9. Un procédé selon la revendication 7 ou la revendication 8,
caractérisé en ce que la culture est effectuée dans un réacteur du type colonne avec une perfusion de bouillon de culture.

10. Un procédé selon la revendication 7 ou la revendication 8,
caractérisé en ce que la culture est effectuée dans des bouteilles à rouleau.

**Patentansprüche**

4

1. Packmaterialelement für die Massenanzucht von anhaftenden Zellen, dadurch gekennzeichnet, daß das Element aus Kunststoffmaterial einen Abschnitt mit einer um die Längsachse verdrehten ebenen Fläche oder so ein Band aufweist.

2. Element gemäß Anspruch 1, das die Gestalt eines Bandbereichs besitzt, der in die Form einer planaren linearen Helix verdreht ist.

3. Element gemäß Anspruch 1, das die Gestalt eines verdrehten Quadrats besitzt.

4. Element gemäß einem der vorangegangenen Ansprüche, dadurch gekennzeichnet, daß das Kunststoffmaterial transparent ist.

5. Element gemäß einem der vorangegangenen Ansprüche, dadurch gekennzeichnet, daß das Kunststoffmaterial Polystyrol, Polypropylen oder Polyester ist.

6. Element gemäß einem der vorangegangenen Ansprüche, dadurch gekennzeichnet, daß das Element zwischen 0,05 mm und 0,25 mm dick ist und eine Länge von mindestens 2 mm und eine Breite zwischen 1 mm und 5 mm besitzt.

7. Verfahren zur Massenanzucht von anhaftenden Zellen in einem Packmaterialelemente enthaltenden Behälter, dadurch gekennzeichnet, daß die Elemente gemäß einem der vorangegangenen Ansprüche sind.

8. Verfahren zur Anzucht gemäß Anspruch 7, dadurch gekennzeichnet, daß die Packelemente willkürlich gepackt sind.

9. Verfahren gemäß Anspruch 7 oder 8, dadurch gekennzeichnet, daß die Zucht in einem säulenartigen Reaktor unter Mediumdurchfluß erfolgt.

10. Verfahren gemäß Anspruch 7 oder 8, dadurch gekennzeichnet, daß die Zucht in Rollerflaschen erfolgt.

FIG . 1

DAYS

FIG . 2

FIG.3

FIG. 4

FIG. 5B

FIG. 5A